# EUROPEAN PATENT APPLICATION

(11) **EP 4 775 993 A1**
(43) Date of publication of application: **15.07.2026**
(21) Application number: 25382013.8
(22) Date of filing: 13.01.2025
(51) Int. Cl.: G01N 33/68

(54) **ASSAY FOR TDP-43 DETERMINATION**

(71) Applicant: Fundación Instituto Madrileño de Estudios Avanzados en Nanociencia (IMDEA-Nanociencia), 28049 Madrid Madrid (ES)
(72) Inventor: PALOMO RUIZ, María del Valle, 28049 Madrid (ES); FERNÁNDEZ GÓMEZ, Paula, 28049 Madrid (ES)
(74) Representative: Hoffmann Eitle

(57) **Abstract**

The present invention is related to the field of medical diagnosis. In particular, we disclose the development and validation of a novel assay for TDP-43 determination, which is highly sensitive, can quantify TDP-43 levels in human lymphocytes and is useful in the diagnosis of ALS and other related TDP-43 proteinopathies.

## Description

### TECHNICAL FIELD

The present invention is related to the field of medical diagnosis. In particular, we disclose the development and validation of a novel assay for TDP-43 determination, which is highly sensitive, can quantify TDP-43 levels in human lymphocytes and is useful in the diagnosis of ALS and other related TDP-43 proteinopathies.

### BACKGROUND ART

TAR DNA-binding protein 43 (TDP-43) is a 414-amino acid nuclear protein belonging to the heterogeneous ribonucleoprotein (hnRNP) family and has been identified as a major component of ubiquitin-positive inclusions found in degenerating neurons and glial cells of patients with frontotemporal lobar degeneration (FTLD) and amyotrophic lateral sclerosis (ALS). Since the systemic degeneration of motor neurons in ALS causes progressive muscle atrophy, paralysis, and eventual death within 2-5 years, early and accurate diagnosis would be valuable for patients, their families, and doctors. However, the early diagnosis of ALS is challenging because differentiating it from other diseases that mimic ALS based only on clinical symptoms and signs is difficult. To address this problem, molecular biomarkers, especially fluid biomarkers that are safe, inexpensive, and widely accessible, are necessary. Thus far, neurofilament light chain (NfL) is the most studied fluid biomarker for ALS, and its levels in the cerebrospinal fluid (CSF) and serum/plasma have been reported to increase in patients with ALS compared with those in controls. Moreover, they were associated with poor outcomes. Despite these promising results, changes in NfL levels in the CSF and blood are not specific to ALS; therefore, other fluid biomarkers that reflect pathognomonic pathologies of ALS, such as TDP-43 accumulation in neurons, are still needed.

Thus far, some studies have used conventional enzyme-linked immunosorbent assay (ELISA) techniques that reported the potential usefulness of TDP-43 levels in the CSF and plasma as a biochemical biomarker to support ALS diagnosis. Although these studies have identified high TDP-43 levels in the CSF or plasma from patients with ALS compared with controls, the absolute concentrations of TDP-43 immunoassays are inconsistent for measuring this protein in biofluids. This is primarily caused by those studies that used ELISA techniques that did not have sufficient sensitivity to correctly measure small amounts of TDP-43 in human biofluids. To overcome this insufficient sensitivity, authors have reported using different techniques, but none have disclosed a useful method to identified high TDP-43 levels in non-immortalized lymphocytes isolated from subjects at risk of suffering from

In this invention, we report the development and validation of a novel assay for TDP-43 determination, which is highly sensitive, can quantify TDP-43 levels in human lymphocytes and is useful in the diagnosis of ALS and other related TDP-43 proteinopathies.

### BRIEF DESCRIPTION OF THE FIGURES

**Figure 1****-** Flow cytometry analysis of TDP-43 and pTDP-43 in ALS patients lymphoblasts and healthy controls. (A) Single patient analysis of TDP-43 using QD605 and QD655 secondary antibody conjugate and QD655 streptavidin conjugates. (B) TDP-43 quantification using QD655 and Alexa488 in healthy controls (red) and ALS patients (green). (*p<0.05, **p<0.01)
**Figure 2****-** Flow cytometry quantification of ALS lymphocytes. (A) Single-patient quantification comparing controls and ALS patients representing only QD MFI. (B) Quantification comparing controls and ALS patients using the QD/Alexa TDP-43 MFI ratio.
**Figure 3****-** Flow cytometry quantification of FTLD lymphocytes. Quantification comparing controls and FTLD patients using the QD/Alexa TDP-43 MFI ratio.

### DETAILED DESCRIPTION OF THE INVENTION

In the present invention, we herein confront the problem of developing a novel assay for TDP-43 determination, which is highly sensitive, can quantify TDP-43 levels in human non-immortalized lymphocytes and is useful in the diagnosis of ALS and other related TDP-43 proteinopathies.

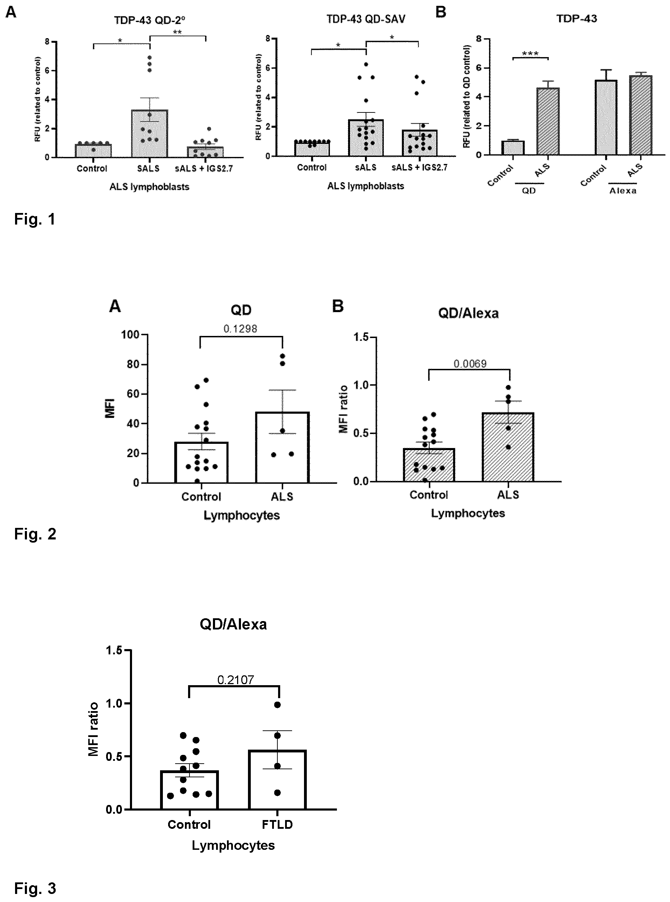

In example 1, we observed that in an established cell line (immortalized lymphocytes) derived from healthy individuals and ALS patients, the selective cytosolic labelling by using quantum dots, resulted in significant differences in the TDP-43 levels between ALS patients and healthy individuals or treated ALS patients' samples (Figure 1A). However, if instead of using a selective cytosolic labelling such as by using quantum dots, TDP-43 levels are determined by using traditional fluorophores without the ability of selective cytosolic labelling (such as Alexa), healthy individuals and ALS patients present similar values (Figure 1B).

Based on the above, we decided to reproduce the results from example 1 but in fresh lymphocytes. However, when working with lymphocytes directly obtained from a blood draw, we observed highly variable results with both QD and Alexa staining values, it was thus not possible to correctly strategy ALS patients from healthy individuals (Figure 2A). To resolve this problem, instead of using absolute values we used the ratio QD/Alexa as a measure of TDP-43 pathology. Remarkably, by using said ratio, ALS patients and healthy controls were correctly grouped (Figure 2B).

In conclusion, this novel assay for TDP-43 determination could be used not only for correctly classifying ALS patients from healthy controls but specifically to measure TDP-43 pathology burden and monitor pharmacological activity at the molecular level from a blood draw.

Therefore, a first aspect of the invention refers to a method for screening or identifying subjects at risk of suffering or having a TDP-43 proteinopathy, the method comprising calculating a ratio of the cytoplasmic concentration to the total cellular concentration of TDP-43 in mononuclear immunological cells (from herein after "cell sample") isolated from a blood sample of the subject; comparing the calculated ratio to one or more predefined decision thresholds to classify the subject into at least one category indicative of a higher or lower likelihood of disease presence; and generating a risk stratification output that specifies whether the subject falls within a category associated with an increased or decreased likelihood of having the disease (TDP-43 proteinopathy).

TDP-43 proteinopathies consist of a group of neurodegenerative diseases defined by the pathological presence of misfolded proteins and insoluble deposits of the transactive response DNA-binding protein of 43 kDa (TDP-43) in the central nervous system (CNS), in association with progressive neuronal loss and gliosis. Preferred TDP-43 proteinopathies are selected from the list consisting of amyotrophic lateral sclerosis, frontotemporal lobar degeneration or limbic-predominant age-related TDP-43 encephalopathy. Preferably, amyotrophic lateral sclerosis.

The term "cytoplasmic concentration," as used herein, refers to the measurable quantity of TDP-43 localized within the cytoplasmic compartment of a mononuclear immunological cell in the cell sample, expressed per unit volume or mass of the cytoplasm.

The term "total cellular concentration," as used herein, refers to the aggregate measurable quantity of TDP-43 present throughout the entirety of the mononuclear immunological cell in the cell sample, encompassing all cellular compartments, expressed per unit volume or mass of the cell.

The term "subject," as used herein, refers to a living mammal, including but not limited to humans, from whom a biological sample, such as a blood sample, is obtained for the purpose of evaluating biomarker concentrations, determining the likelihood of disease presence, or performing risk stratification. Preferably, the subject is a human being, encompassing individuals of any age, gender, or health status.

The term "predefined decision thresholds," as used herein, refers to one or more predetermined quantitative or qualitative criteria established based on empirical data, statistical models, or expert consensus, against which the calculated ratio of cytoplasmic concentration to total cellular concentration of the biomarker is compared to categorize the subject's likelihood of disease presence.

The term "category," as used herein, refers to a predefined classification group into which a subject is placed based on the comparison of biomarker concentration ratios to the predefined decision thresholds, such categories being indicative of distinct levels of likelihood of disease presence.

The term "higher or lower likelihood of disease presence," as used herein, refers to the relative probability that a subject either currently has, or is at increased or decreased risk of developing, a specific disease, condition, or pathological state. This probability is quantitatively or qualitatively determined by comparing a calculated biomarker value or ratio, such as the cytoplasmic concentration-to-total cellular concentration ratio of a biomarker, to one or more predefined decision thresholds.

The term "risk stratification output," as used herein, refers to a generated result or report, derived from the classification of the subject based on biomarker analysis, which specifies whether the subject belongs to a category associated with an increased or decreased likelihood of disease presence, potentially guiding clinical decisions or further diagnostic evaluation.

Mononuclear immunological cells, as used herein, are a subclass of immune cells characterized by the presence of a singular, non-lobulated nucleus, distinct from polymorphonuclear cells. These cells encompass monocytes and lymphocytes, which include subsets such as T lymphocytes, B lymphocytes, and natural killer (NK) cells. Mononuclear immunological cells are integral to immune system function, encompassing roles in antigen presentation, cytokine production, phagocytosis, antibody generation, and cellular immunity. In a specified embodiment, mononuclear immunological cells include monocytes, which are precursors to macrophages and dendritic cells, functioning in the innate immune response and antigen presentation to adaptive immune system components. Alternatively, these cells may include lymphocytes, which mediate adaptive immunity through mechanisms such as cell-mediated cytotoxicity, humoral immunity via antibody secretion, and immune regulation. In the context of the present invention, mononuclear immunological cells isolated from a blood sample of the subject are preferably lymphocytes. The lymphocytes are primary, non-immortalized cells obtained directly from the subject's blood via standard blood collection techniques, such as venipuncture. These lymphocytes are naturally occurring cells that have not undergone any artificial immortalization processes or genetic modifications to render them capable of indefinite proliferation. Instead, they represent the subject's authentic immune profile at the time of collection. Importantly, the lymphocytes are not derived from established or artificially maintained cell lines but are freshly isolated from the subject's peripheral blood following standard separation techniques, such as density gradient centrifugation, to specifically enrich for mononuclear cell populations.

A primary antibody that specifically binds the target protein refers to an antibody that directly recognizes and binds to a specific epitope on the target protein with high affinity. Nowadays, antibodies exist to detect TDP-43 and phosphorylated TDP-43. In the present invention, the primary antibody can bind TDP-43 and/or phosphorylated TDP-43. Moreover, more than one primary antibody can be used.

It is noted that in the context of the present invention, the terms "subject" and "individual" are interchangeable.

In a preferred embodiment, the method of the first aspect of the invention comprises the steps of:
a. determining the cytoplasmic concentration and the total cellular concentration of TDP-43 in the mononuclear immunological cells (cell sample) isolated from the subject;
b. calculating a ratio of the cytoplasmic concentration to the total cellular concentration of TDP-43;
c. comparing the calculated ratio to one or more predefined decision thresholds to classify the individual into at least one category indicative of a higher or lower likelihood of disease presence; and
d. generating a risk stratification output that specifies whether the individual falls within a category associated with an increased or decreased likelihood of having the disease.

In another preferred embodiment of the first aspect of the invention, optionally in combination with any previous or subsequent preferred embodiment of the invention, the TDP-43 proteinopathy is selected from the list consisting of amyotrophic lateral sclerosis, frontotemporal lobar degeneration or limbic-predominant age-related TDP-43 encephalopathy. Preferably, the TDP-43 proteinopathy is amyotrophic lateral sclerosis.

In another preferred embodiment of the first aspect of the invention, optionally in combination with any previous or subsequent preferred embodiment of the invention, the total cellular concentration and the cytoplasmic concentration of the TDP-43 protein are determined by an immunoassay selected from the group consisting of:
a. Western blot,
b. enzyme-linked immunosorbent assay (ELISA),
c. immunofluorescence, and
d. flow cytometry or imaging flow cytometry.

In another preferred embodiment of the first aspect of the invention, optionally in combination with any previous or subsequent preferred embodiments of the invention, the cytoplasmic concentration of the TDP-43 protein is determined by incubating the cell sample with a primary antibody that specifically binds the TDP-43 protein and incubating the cell sample with a secondary antibody conjugated to one or more Quantum Dots (QD), under conditions sufficient to form a complex with the primary antibody; and analyzing the cell sample to detect signals from QDs, thereby determining the cytoplasmic concentration of the TDP-43 protein. Preferably, but not limited to, the analysis is performed using flow cytometry or any other similar technique capable of detecting and quantifying the signals from QDs.

In another preferred embodiment of the first aspect of the invention, optionally in combination with any previous or subsequent preferred embodiment of the invention, the cytoplasmic concentration of the biomarker is determined by incubating the cell sample with a primary antibody that specifically binds the TDP-43 protein and incubating the cell sample with a secondary antibody and one or more quantum dots, wherein the secondary antibody specifically binds to the one or more QDs under conditions sufficient to first form a complex between the primary and secondary antibodies and second through a high-affinity binding interaction between the secondary antibody and the QDs; and analyzing the cell sample to detect signals from QDs, thereby determining the cytoplasmic concentration of the TDP-43 protein.

High-affinity binding interactions between a secondary antibody and a fluorophore or a QD, can occur through specific molecular recognition and binding mechanisms under conditions sufficient to facilitate such interactions. For instance, in one embodiment, the secondary antibody is conjugated to a binding moiety (e.g., biotin) capable of engaging in a high-affinity interaction with a complementary molecule (e.g., streptavidin).

The high-affinity interaction between biotin and streptavidin is exemplary of such a mechanism. Biotin, a small molecule structurally designed to fit with high specificity into the binding pocket of streptavidin, forms a stable non-covalent complex characterized by a dissociation constant (Kd) in the range of ~10⁻¹⁵ M. This interaction is stabilized by multiple molecular forces, including hydrogen bonding, van der Waals forces, and electrostatic interactions. In certain embodiments, a biotin-conjugated secondary antibody first forms a complex with a primary antibody bound to the target molecule. Subsequently, this biotin-labeled secondary antibody binds to streptavidin through the high-affinity interaction, thereby forming a stable and specific multi-component complex. The resulting complex remains intact under stringent conditions, facilitating its use in applications requiring robust molecular interactions, such as immunoassays, purification, or detection methods.

In another preferred embodiment of the first aspect of the invention, optionally in combination with any previous or subsequent preferred embodiment of the invention, the total cellular concentration of the TDP-43 protein is determined by incubating the cell sample with a primary antibody that specifically binds the TDP-43 protein and incubating the cell sample with a secondary antibody conjugated to a fluorophore (F), distinct from a QD and capable of labelling the nucleus and the cytoplasm of the cells, under conditions sufficient to form a complex with the primary antibody; and analyzing the cell sample to detect signals from F, thereby determining the total cellular concentration of the TDP-43 protein.

In another preferred embodiment of the first aspect of the invention, optionally in combination with any previous or subsequent preferred embodiment of the invention, the total cellular concentration of the TDP-43 protein is determined by incubating the cell sample with a primary antibody that specifically binds the TDP-43 protein and incubating the cell sample with a secondary antibody and one or more fluorophores (F), distinct from a QD and capable of labelling the nucleus and the cytoplasm of the cells, under conditions sufficient to first form a complex between the primary and secondary antibodies and second through a high-affinity binding interaction between the secondary antibody and the Fs; and analyzing the cell sample to detect signals from Fs, thereby determining the total cellular concentration of the TDP-43 protein.

In another preferred embodiment of the first aspect of the invention, optionally in combination with any previous or subsequent preferred embodiment of the invention, the cytoplasmic concentration of the TDP-43 protein is determined:
- by incubating the cell sample with a primary antibody that specifically binds the TDP-43 protein and incubating the cell sample with a secondary antibody conjugated to one or more Quantum Dots (QD), under conditions sufficient to form a complex with the primary antibody; and analysing the cell sample to detect signals from QDs, thereby determining the cytoplasmic concentration of the TDP-43 protein. Preferably, but not limited to, the analysis is performed using flow cytometry or any other similar technique capable of detecting and quantifying the signals from QDs; and/or
- by incubating the cell sample with a primary antibody that specifically binds the TDP-43 protein and incubating the cell sample with a secondary antibody and simultaneously or subsequently with one or more quantum dots, wherein the secondary antibody specifically binds to the one or more QDs under conditions sufficient to first form a complex between the primary and secondary antibodies and second through a high-affinity binding interaction between the secondary antibody and the QDs; and analysing the cell sample to detect signals from QDs, thereby determining the cytoplasmic concentration of the TDP-43 protein. Preferably, but not limited to, the analysis is performed using flow cytometry or any other similar technique capable of detecting and quantifying the signals from QDs;
   and the total cellular concentration of the TDP-43 protein is determined:
- by incubating the cell sample with a primary antibody that specifically binds the TDP-43 protein and incubating the cell sample with a secondary antibody conjugated to a fluorophore (F), distinct from a QD and capable of labelling the nucleus and the cytoplasm of the cells, under conditions sufficient to form a complex with the primary antibody; and analysing the cell sample to detect signals from F, thereby determining the total cellular concentration of the TDP-43 protein. Preferably, but not limited to, the analysis is performed using flow cytometry or any other similar technique capable of detecting and quantifying the signals from F; and/or
- by incubating the cell sample with a primary antibody that specifically binds the TDP-43 protein and incubating the cell sample with a secondary antibody and simultaneously or subsequently with one or more fluorophores (F), distinct from a QD and capable of labelling the nucleus and the cytoplasm of the cells, under conditions sufficient to first form a complex between the primary and secondary antibodies and second through a high-affinity binding interaction between the secondary antibody and the Fs; and analysing the cell sample to detect signals from Fs, thereby determining the total cellular concentration of the TDP-43 protein. Preferably, but not limited to, the analysis is performed using flow cytometry or any other similar technique capable of detecting and quantifying the signals from F.

The above methodology of determining the cytoplasmic concentration and the total cellular concentration of the TDP-43 protein shall be herein referred to as "the specific methodology of the invention".

In another preferred embodiment of the first aspect of the invention, optionally in combination with any previous or subsequent preferred embodiment of the invention, the predetermined threshold is selected based on statistical analysis of TDP-43 ratios obtained from a reference or healthy control population, wherein if in the comparing step the ratio result is higher than the decision threshold the subject shall be categorize as an individual into at least the higher-risk group of suffering from a TDP-43 proteinopathy (an increased likelihood of having the disease) otherwise the subject shall be categorized as a lower-risk group (a decrease likelihood of having the disease).

In another preferred embodiment of the first aspect of the invention, optionally in combination with any previous or subsequent preferred embodiment of the invention, the cytoplasmic concentration and the total cellular concentration of the TDP-43 protein are determined as indicated in the specific methodology of the invention, and the predetermined threshold is selected based on statistical analysis of TDP-43 ratios obtained from a reference or control population, wherein if in the comparing step the ratio result is higher than the decision threshold the subject shall be categorize as a higher-risk group (an increased likelihood of having the disease) of suffering from a TDP-43 proteinopathy otherwise the subject shall be categorized as a lower-risk group (a decrease likelihood of having the disease); and the TDP-43 proteinopathy is preferably amyotrophic lateral sclerosis.

Quantum dots (QDs), as used herein, are nanocrystals of semiconducting material possessing quantum mechanical characteristics with capability to get conjugated with drug moieties. The particle size of QDs varies from 2 to 10 nm and can radiate a wide range of colours depending upon their size. Their wide and diverse usage of QDs across the world is due to their adaptable properties like large quantum yield, photostability, and adjustable emission spectrum. QDs are nanomaterials with inherent electrical characteristics that can be used as drug carrier vehicle and as a diagnostic in the field of nanomedicine. Typically, commercially available quantum dots are named for their peak emission wavelength (i.e., 525, 565, 585, 605, and 655 nm). In one embodiment, any quantum dot such as QD525, 565, 585, 605, or 655 nm, can be used in the present invention.

In another preferred embodiment of the first aspect of the invention, optionally in combination with any previous or subsequent preferred embodiment of the invention, the QD and the fluorophore (F) are selected from different emission spectra suitable for multicolor flow cytometry, allowing simultaneous detection of multiple signals.

In another preferred embodiment of the first aspect of the invention, optionally in combination with any previous or subsequent preferred embodiment of the invention, the QD has a peak emission wavelength of 525, 565, 585, 605, or 655 nm such as Qdot 655 and the fluorophore (F) is preferably Alexa fluor 488.

In another preferred embodiment of the first aspect of the invention, optionally in combination with any previous or subsequent preferred embodiment of the invention, the method further comprises selecting or adjusting a therapeutic regimen for the subject based on the classification, wherein subjects classified above a certain ratio threshold receive a different therapy than subjects classified below said threshold.

In another preferred embodiment of the first aspect of the invention, optionally in combination with any previous or subsequent preferred embodiment of the invention, calculating the ratio and classifying the subject is performed by a computer-implemented algorithm that processes input data from the measurements in steps (b) and (c) and outputs an indication of the subject's classification.

In another preferred embodiment of the first aspect of the invention, optionally in combination with any previous or subsequent preferred embodiment of the invention, the method further comprises utilizing a kit or system configured to:
- isolate cytoplasmic fractions from the sample,
- detect or quantify the biomarker in both the cytoplasmic fraction and the whole-cell fraction, and
- provide instructions for comparing the ratio to the predetermined threshold to classify the subject.

The following example is merely for illustrative purposes and does not limit the present invention.

### EXAMPLE

**Example 1:** Characterization of TDP-43 pathology in lymphoblasts from ALS patients using QDs, both secondary antibody-conjugated QDs and streptavidin-conjugated QDs.

In this case, we are using an established cell line (immortalized lymphocytes) derived from healthy individuals and ALS patients. Due to their selective cytosolic labelling, there are significant differences between Controls, ALS patients, and treated ALS patients' samples (Figure 1A). We observe that traditional fluorophores without ability for selective cytosolic labelling (such as Alexa) present similar values in high contrast to QDs that classifies correctly the samples (Figure 1B).

**Example 2:** Characterization of TDP-43 pathology in lymphocytes from ALS patients using streptavidin-conjugated QDs, and Alexa Fluor 488.

In this case, the original idea was to reproduce the results from example 1 but in fresh lymphocytes. However, when working with lymphocytes directly obtained from a blood draw, we observed highly variable results with both QD and Alexa staining values, and a correct classification cannot be done in a similar way to Example 1 (Figure 2A). This challenge has been solved using the ratio QD/Alexa as a measure of TDP-43 pathology, that is performed in each patient data. ALS patients and healthy controls are correctly grouped with this correction (Figure 2B). We believe this methodology could be used not only for correctly classifying ALS patients from healthy controls but specifically to measure TDP-43 pathology burden and monitor pharmacological activity at the molecular level from a blood draw. It could be useful for companies developing TDP-43 targeting therapies to show in vivo target engagement and in vivo efficacy (PoC).

**Example 3:** Characterization of TDP-43 pathology in lymphocytes from FTLD patients using streptavidin-conjugated QDs, and Alexa Fluor 488.

In this case, we have used the method to measure TDP-43 pathology in lymphocytes from FTLD patients. While the ratio has not given significant differences from healthy controls, given that FTLD pathology is roughly 50% based on tau pathology and 50% on TDP-43 pathology, it could help to classify patients according to their molecular profile that could be extremely useful to select effective drug for these patients.

### MATERIALS AND METHODS

### 1. Flow cytometry protocol

1.1. Fresh lymphocytes are extracted from the blood of the patients and obtained by Ficoll-Paque.
Cells are fixed with 4% PFA in PBS1X for 15 min at 37°C. Subsequently, they are permeabilized with 0.5% Tween 20 in PBS1X for 25 min, after which the blocking step is performed by adding 5% NGS for 30 min.
Cells are incubated with primary antibody (c-terminal TDP-43) for 1h at 37 °C used at 1:100 dilution in 5% NGS and the cells are incubated overnight at 4°. For the negative control, IgG isotype primary antibody is employed.

### 1.2. Streptavidin Quantum Dots (QD-sav)

Subsequent to primary antibody incubation, cells are incubated with biotinylated antimouse or anti-rabbit secondary antibody at a 1:500 dilution in 5% NGS for 1 h at room temperature. The samples are then washed twice with PBS1X and incubated with QD655-sav at 1:100 dilution in 5% NGS at room temperature for 1 h followed by a wash with PBS1X.

### 1.3. Quantum Dots Secondary Antibody (QD-Ab2)

Subsequent to primary antibody incubation, cells are incubated with QD655 conjugated with secondary antibody at a 1:100 dilution in 5% NGS at room temperature for 1 h followed by a PBS1X wash.

### 1.4. Alexa Fluor 488 (A488) Secondary Antibody

Subsequent to primary antibody incubation, cells are incubated with Alexa Fluor 488 conjugated with secondary antibody at a 1:500 dilution in 5% NGS at room temperature for 1 h and washed with PBS1X.

### 2. Data Acquisition and analysis

Cell suspension is analysed using a Cytoflex flow cytometer (Beckman-Coulter) and 405 nm laser as excitation source for QDs and 488 nm laser for A488 were used. A minimum of 1 million cells are acquired unless limited by the total number of isolated cells. Sample gating is performed by taking the lymphoblasts population based on the FSC (cell size) and SSC (complexity). Cytexpert (Beckman-Coulter) software is used to analyze all data. Immortalized lymphoblast data is analysed by calculating the relative fluorescence unit (RFU) against control's mean for each day of measurement. Mean fluorescence intensity (MFI) ratio among QD intensity and Alexa488 values of each condition is done for Patients' lymphocytes data. GraphPad Prism 8 software is used for data analysis. Firstly, normality test is done for each data and, as the data followed a normal distribution, a one-way ANOVA analysis was employed. Statistically differences were analysed using Bonferroni's analysis. Data is represented by the mean±SEM.

### 3. Patient data:

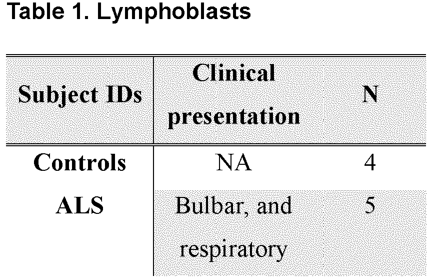

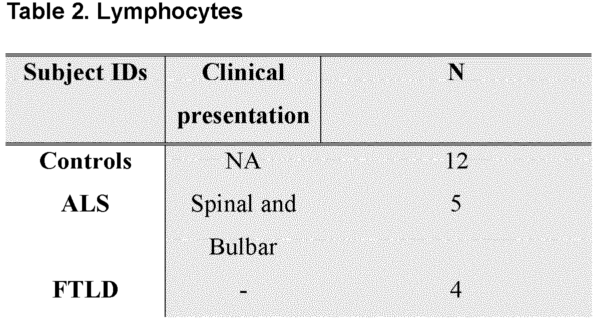

**Table 3. Reagents table**

| | | |
|---|---|---|
| | | |

| **REAGENT OR RESOURCE** | **SOURCE** | **CAT. N°** |
|---|---|---|
| **Antibodies** | | |
| TDP-43 c-terminal | Proteintech | 67345-1-Ig |
| IgG isotype | Thermo Fisher | 10400C |
| Biotinylated secondary antibody rabbit/mouse | VectorLabs | BA-1000/BA-2000 |
| | | |

| **Fluorophores** | | |
|---|---|---|
| QD565 secondary antibody | Thermo Fisher | Q11032MP |
| QD605 secondary antibody | Thermo Fisher | Q11402MP |
| QD655 secondary antibody | Thermo Fisher | Q11422MP |
| QD705 secondary antibody | Thermo Fisher | Q11461MP |
| QD565 streptavidin | Thermo Fisher | Q10133MP |
| QD605 streptavidin | Thermo Fisher | Q10103MP |
| QD655 streptavidin | Thermo Fisher | Q10123MP |
| QD705 streptavidin | Thermo Fisher | Q10163MP |
| Alexa Fluor 488 secondary antibody | Thermo Fisher | A-10680 |
| | | |

| **Reagents** | | |
|---|---|---|
| RPMI-1640 medium | VWR | 392-0426P |
| 10% FBS | Gibco | 16140071 |
| 1% penicillin/streptomycin | Gibco | 15140148 |
| Paraformaldehyde | Thermo Fisher | 047317.9M |
| Triton X-100 | Sigma Aldrich | A16046.AE |
| Phosphate buffered saline (PBS) | | |
| Normal Goat Serum (NGS) | Gibco | 10098792 |
| Avidin/Biotin blocking kit | Thermo Fisher | R37627 |
| Histomount mounting medium | Sigma Aldrich | F4680 |
| Tween20 | Thermo Fisher | J20605.AP |
| Gelatin | Thermo Fisher | 410875000 |
| Poly-D-lysine | Fisher Scientific | 16021412 |
| **Ficoll^{®} Paque Plus** | Sigma Aldrich | GE17-1440-02 |
| | | |

## Claims

1. A method for screening or identifying subjects at risk of suffering or having a TDP-43 proteinopathy, the method comprising calculating a ratio of the cytoplasmic concentration to the total cellular concentration of TDP-43 in mononuclear immunological cells (from herein after "cell sample") isolated from a blood sample of the subject; comparing the calculated ratio to one or more predefined decision thresholds to classify the subject into at least one category indicative of a higher or lower likelihood of disease presence; and generating a risk stratification output that specifies whether the subject falls within a category associated with an increased or decreased likelihood of having the disease; wherein the mononuclear immunological cells are primary, non-immortalized cells, obtained directly from the subject's peripheral blood, excluding any artificially immortalized or cultured cell lines.

2. The method of claim 1, wherein the method comprises:
a. determining the cytoplasmic concentration and the total cellular concentration of TDP-43 in the mononuclear immunological cells (cell sample) isolated from the subject;
b. calculating a ratio of the cytoplasmic concentration to the total cellular concentration of TDP-43;
c. comparing the calculated ratio to one or more predefined decision thresholds to classify the individual into at least one category indicative of a higher or lower likelihood of disease presence; and
d. generating a risk stratification output that specifies whether the subject falls within a category associated with an increased or decreased likelihood of having the disease.

3. The method of claim 1 or 2, wherein the TDP-43 proteinopathy is selected from the list consisting of amyotrophic lateral sclerosis, frontotemporal lobar degeneration and limbic-predominant age-related TDP-43 encephalopathy.

4. The method of any one of claims 1 to 3, wherein the total cellular concentration and the cytoplasmic concentration of the TDP-43 protein are determined by an immunoassay selected from the group consisting of:
a. Western blot,
b. enzyme-linked immunosorbent assay (ELISA),
c. immunofluorescence, and
d. flow cytometry or imaging flow cytometry.

5. The method of claim 1 to 3, wherein the cytoplasmic concentration of the TDP-43 protein is determined by incubating the cell sample with a primary antibody that specifically binds the TDP-43 protein (TAR DNA-binding protein 43 (TDP-43) and/or phosphorylated TDP-43 (pTDP-43)) and incubating the cell sample with a secondary antibody conjugated to one or more Quantum Dots (QD) under conditions sufficient to form a complex with the primary antibody; and analysing the cell sample to detect signals from QDs, thereby determining the cytoplasmic concentration of the TDP-43 protein.

6. The method of claim 1 to 3, wherein the cytoplasmic concentration of the biomarker is determined by incubating the cell sample with a primary antibody that specifically binds the TDP-43 protein (TAR DNA-binding protein 43 (TDP-43) and/or phosphorylated TDP-43 (pTDP-43)) and incubating the cell sample with a secondary antibody and simultaneously or subsequently with one or more quantum dots, wherein the secondary antibody specifically binds to the one or more QDs under conditions sufficient to first form a complex between the primary and secondary antibodies and second through a high-affinity binding interaction between the secondary antibody and the QDs; and analysing the cell sample to detect signals from QDs, thereby determining the cytoplasmic concentration of the TDP-43 protein.

7. The method of claim 1 to 6, wherein the total cellular concentration of the TDP-43 protein is determined by incubating the cell sample with a primary antibody that specifically binds the TDP-43 protein (TAR DNA-binding protein 43 (TDP-43) and/or phosphorylated TDP-43 (pTDP-43)) and incubating the cell sample with a secondary antibody conjugated to a fluorophore (F), distinct from a QD and capable of labelling the nucleus and the cytoplasm of the cells, under conditions sufficient to form a complex with the primary antibody; and analysing the cell sample to detect signals from F, thereby determining the total cellular concentration of the TDP-43 protein.

8. The method of claim 1 to 6, wherein the total cellular concentration of the TDP-43 protein is determined by incubating the cell sample with a primary antibody that specifically binds the TDP-43 protein (TAR DNA-binding protein 43 (TDP-43) and/or phosphorylated TDP-43 (pTDP-43)) and incubating the cell sample with a secondary antibody and one or more fluorophores (F), distinct from a QD and capable of labelling the nucleus and the cytoplasm of the cells, under conditions sufficient to first form a complex between the primary and secondary antibodies and second through a high-affinity binding interaction between the secondary antibody and the Fs; and analysing the cell sample to detect signals from Fs, thereby determining the total cellular concentration of the TDP-43 protein.

9. The method according to claims 5 to 8, wherein the cytoplasmic concentration of the TDP-43 protein is determined according to claim 5 or 6 and the total cellular concentration of the TDP-43 protein is determined according to claim 7 or 8.

10. The method of any one of claims 5 to 7, wherein the QD and the fluorophore (F) are selected from different emission spectra suitable for multicolor flow cytometry, allowing simultaneous detection of multiple signals.

11. The method of any one of claims 1 to 8, wherein the predetermined threshold is selected based on statistical analysis of TDP-43 ratios obtained from a reference or control population, wherein if in the comparing step the ratio result is higher than the decision threshold the subject shall be categorize as an individual into at least the higher-risk group of suffering from a TDP-43 proteinopathy otherwise the subject shall be categorized as a lower-risk group.

12. The method according to claim 2, wherein the cytoplasmic concentration of the TDP-43 protein is determined according to claim 5 or 6 and the total cellular concentration of the TDP-43 protein is determined according to claim 7 or 8; wherein the predetermined threshold is selected based on statistical analysis of TDP-43 ratios obtained from a reference or control population, wherein if in the comparing step the ratio result is higher than the decision threshold the subject shall be categorize as a higher-risk group of suffering from a TDP-43 proteinopathy otherwise the subject shall be categorized as a lower-risk group; and wherein the TDP-43 proteinopathy is amyotrophic lateral sclerosis.

13. The method according to claim 12, wherein the QD is Qdot 655 and the fluorophore (F) is Alexa fluor 488.

14. The method of any one of claims 1 to 13, further comprising selecting or adjusting a therapeutic regimen for the subject based on the classification, wherein subjects classified above a certain ratio threshold receive a different therapy than subjects classified below said threshold.

15. The method of any one of claims 1 to 14, wherein calculating the ratio and classifying the subject is performed by a computer-implemented algorithm that processes input data from the measurements in steps (b) and (c) and outputs an indication of the subject's classification.

16. The method of any one of claims 1 to 15, further comprising utilizing a kit or system configured to:
a. isolate cytoplasmic fractions from the sample,
b. detect or quantify the biomarker in both the cytoplasmic fraction and the whole-cell fraction, and
c. provide instructions for comparing the ratio to the predetermined threshold to classify the subject.
